# EUROPEAN PATENT SPECIFICATION

(11) **EP 1 139 166 B1**
(45) Date of publication and mention of the grant of the patent: **05.01.2005**
(21) Application number: 01200918.9
(22) Date of filing: 12.03.2001
(51) Int. Cl.: G03C 3/00, G03B 42/04

(54) **Comfort-enhancing intraoral dental radiographic film packet and method for forming same**
Den Komfort verbessernde intraorale Dentalröntgenfilmpackung und Verfahren zur Herstellung derselben
Paquet film dental radiographique intraorale augmentent le confort et procédé pour produire le-même

(30) Priority: 24.03.2000 US 534516; 24.03.2000 US 534368; 24.03.2000 US 533867; 24.03.2000 US 534372; 24.03.2000 US 534393; 24.03.2000 US 534370; 24.03.2000 US 533868; 24.03.2000 US 534392
(43) Date of publication of application: 04.10.2001
(73) Proprietor: EASTMAN KODAK COMPANY, Rochester, New York 14650 (US)
(72) Inventor: Resch, Douglas T., Eastman Kodak Company, Rochester, New York 14650-2201 (US); Konte, Bruce W., Eastman Kodak Company, Rochester, New York 14650-2201 (US); Earnhart, Edgar G., Eastman Kodak Company, Rochester, New York 14650-2201 (US); Richter, Edward B., Eastman Kodak Company, Rochester, New York 14650-2201 (US); He, Fugui, Eastman Kodak Company, Rochester, New York 14650-2201 (US); Schwallie, Scott H., Eastman Kodak Company, Rochester, New York 14650-2201 (US); Merz, Gary E., Eastman Kodak Company, Rochester, New York 14650-2201 (US); McGovern, Michael R., Eastman Kodak Company, Rochester, New York 14650-2201 (US); Bacchetta, Richard W., Eastman Kodak Company, Rochester, New York 14650-2201 (US); Allen, David C., Eastman Kodak Company, Rochester, New York 14650-2201 (US)
(74) Representative: Haile, Helen Cynthia

(56) References cited:
- EP-A- 0 564 011
- US-A- 4 791 657
- US-A- 4 922 511
- US-A- 5 170 423

## Description

The present invention relates generally to methods for manufacturing intraoral radiographic film packets with comfort enhancing features, and, in particular, to improvements to intraoral radiographic film packets which allow for ease of handling and positioning in manufacturing operations.

A common problem experienced by people visiting the dentist is the discomfort and pain associated with the taking of dental x-rays caused by the positioning of intraoral radiographic film packets in the patient's mouth. The typical intraoral radiographic film packet includes relatively hard and/or relatively sharp edges that press against and irritate the gums and other oral soft tissue of the person whose teeth are being x-rayed. A variety of intraoral x-ray dental packets are known in the prior art which include features intended to be comfort enhancing. In addition, attempts have been made to create comfort enhancing structures into which intraoral-x-ray dental packets can be inserted prior to placement in the patient's mouth. One example of this type of structure is taught in U.S. Patent No. 5,044,008 titled "Dental Film Cartridge Cushion," by Reginald B. Jackson, August 27, 1991. Jackson utilizes a cartridge cushion comprising a foam sheet sandwich into which the x-ray dental packet is placed for the purpose of cushioning and increasing the comfort to the patient. Jackson requires the manual insertion of the x-ray packet into the cartridge cushion. Thus, Jackson adds significant bulk to the packet and enhances the possibility of triggering a gag reflex action in the patient. Additionally, after the cartridge cushion is removed from the packet, it would be possible to reuse the cartridge cushion which would not be sanitary.

A second example of an add-on structure is taught in U.S. Patent No. 5,285,491 titled "Dental Film Packet," by Muylle et al., February, 8, 1994. Muylle et al. teaches sealing a film pack in an envelope consisting of a pair of thin pockets of injection molded plastic which are sealed with a band of adhesive tape. The envelope has no sharp edges and generally rounded comers. Thus, as with Jackson's device, this device requires manual insertion of the packet, adds significant bulk to the packet, enhances the possibility of triggering a gag reflex in the patient, and can also be reused in a non-sanitary manner.

U.S. Patent No. 1,631,497 titled "Dental X-ray Film Package," by Harry L. Marler, June 7, 1927. Marler teaches a dental x-ray film package wherein a sensitized sheet is sandwiched between two opaque sheets. A heavy band of rubber is stretched about the periphery of the package to hold the package securely together and to provide the light-tight joint.

U.S. Patent No. 1,537,925 titled "Dental X-ray Film Package," by Leonard M. Bolin, May 12, 1925, teaches a dental x-ray film package wherein a pair of film sheets and the cover sheet are inserted into a container. The container consists of a frame including a backing portion in an enlarged continuous beading about the periphery thereof. The beading must be forced away from the backing portion and stretched peripherally in order to insert the film sheets and cover sheet therein. The container thus serves to hold the package together and provide the light-tight seal.

U.S. Patent No. 4,791,657 titled "Intraoral Radiographic Film Packet," by Alan Kirsch et al., December 13, 1988, teaches a dental radiographic film packet which includes soft comers for greater patient comfort. The packet is constructed by removing all material from the comers of a typical dental radiographic film packet with the exception of the film chip. Individual comer covers which are seamless pockets are then added to the four corners of the packet. The comer covers create an airspace at each comer around the edge of the film chip. '

U.S. Patent No. 2,084,092 titled "Dental Film Holder," by Ralph Kenney, June 15, 1937, teaches a dental film holder that is a stretchable vellum rubber plate with integral comer pockets into which an x-ray dental packet may be manually inserted. Kenney's dental film holder is intended to be reusable. The plastic envelope of an intraoral dental radiographic film packet of the type disclosed in U.S. Patent No. 3,443,093 titled "Dental X-ray Packet Having A Uniform Overall Thickness And Method Of Making Same," by Terry N. Lindenmuth et al., May 6, 1969, is typically constructed by joining at the margin thereof two generally parallel sheets of flexible plastic material (typically flexible PVC). Two webs of plastic (which may be referred to as the base and top webs) form the envelopes of the dental film packets. The dental film, lead and opaque sheets are inserted and deposited on the base web and the top web is placed there over. The top and base webs are then sealed at the margin of the each packet to form a continuous web of sealed packets. The continuous web of sealed packets is subsequently die-cut to form a plurality of individual film packets.

Any subsequent operation requiring accurate positioning of the individual film packets such as for the purpose of molding a compliant bead as disclosed in U.S. Patent No. 4,912,740 titled "Intraoral Dental Radiographic Film Packet Improvement," by Elmer W. Liese, Jr., et al., March 27, 1990, becomes very difficult due to several factors including: a) size variation resulting from the die-cutting of flexible material with residual web stress; b) deflection of the marginal area (laminated perimetric edge) of individual film packets (the marginal area does not remain planar after the die-cutting operation); c) the flexible nature of the laminated perimetric edge when placed in a positioning fixture; and d) poor encapsulation of the perimetric edge due to easy and randomized deformation of the marginal area.

From the foregoing it can be seen that many attempts to add a comfort enhancing feature to dental x-ray film packets resulted in structures requiring manual assembly and/or modification of individual film packets in order to receive comfort enhancing structure. Further, such prior art attempts, particularly those seeking to provide the comfort enhancing feature via a frame, have resulted in a significant increase in bulk thereby enhancing the possibility of inducing a gag reflex, and/or also resulted in a structure in which additional manual steps are required for assembly and/or disassembly. Also, the prior art designs fail to provide the features and operations that allow for automated, accurate positioning of individual film packets for automated application of comfort enhancing features thereto that overcome the problems of size variation resulting from the die-cutting of flexible material with residual web stress, deflection of the marginal area of individual film packets, and the flexible nature of the laminated perimetric edge when placed in a positioning fixture for application of a comfort-enhancing perimetric edge bead.

It is therefore, an object of the present invention to provide a semirigid envelope for an intraoral dental radiographic film packet that permits accurate positioning in manufacturing operations such as for the application of a comfort-enhancing bead to the perimetric edges of the marginal area thereby reducing the cutting sensation of the packet by implying a softer, smoother, more rounded feel to the patient's gums, lips and other sensitive tissues of the oral cavity.

It is another object of the present invention to provide a dental film packet wherein the size of the packet is not affected by residual stress in the marginal area after die-cutting a continuous web of sealed dental packets into individualized dental packets.

Yet another object of the present invention is to provide a dental film packet with improved dimensional tolerances wherein the packet is easier to accurately position for subsequent operations such as the application of a comfort-enhancing edge bead.

Still another object of the present invention is to provide a dental film packet that is resistant to deformation when placed against a positioning device for subsequent operations such as the application of a comfort-enhancing edge bead.

The foregoing and numerous other features, objects and advantages of the present invention will become readily apparent upon a review of the detailed description, claims and drawings set forth herein. These features, objects and advantages are accomplished by forming an intraoral dental film packet with an envelope comprised of two outer plastic sheets wherein preferably one of the two outer plastic sheets is a stiffening sheet. The stiffening sheet is more rigid than the typical outer sheets found on prior art dental film packets. The stiffening sheet preferably has a tensile modulus in the range of from 700 to 28,000 Kg/cm². The envelope is formed by joining together the two outer plastic sheets at the marginal or peripheral area to form a perimetric laminated edge that preferably has a tensile modulus in the range of from 400 to 28,000 Kg/cm². By making one of the outer sheets more rigid than the other, the laminated perimetric edge formed by bonding the two outer sheets together is substantially coplanar with the more rigid of the two outer sheets. This resultant rigidity of the film packet in combination with achieving a planar surface on one side of the packet enhances the ability to die cut the packet and maintain outside dimension tolerances. Further, the rigidity obviates the easy deformation of the perimetric edge during subsequent operations in the handling and positioning of individual film packets after the die-cutting operation. This, in turn, allows for more accurate positioning of individual film packets in such subsequent operations. In addition, the planarity of the laminated perimetric edge also aids in achieving full encapsulation by peripheral edge bead. After die cutting there is likely to be some limited deflection (curling) of the laminated perimetric edge away from the plane of the stiffening sheet as a result of the stress exerted by the opposite outer sheet. This limited deflection is still substantially coplanar as compared to the prior art dental film packets. As used herein, the term "substantially coplanar" means that deflection of the laminated perimetric edge away from the plane of the stiffening sheet after die cutting is no more than 20 thousandths of an inch and preferably no more than 10 thousandths of an inch.

In the practice of the method of the present invention individual film packets are transported by a Bernoulli effect apparatus against hard stop reference points. The added rigidity of the laminated perimetric edge and the concomitant improved outside dimensional tolerances achieved therewith allow individual film packets to be positioned by a Bernoulli effect apparatus against hard stop reference points without deformation of the laminated perimetric edge. In such manner, individual film packets can be accurately positioned for subsequent operations including acquisition by a robotic arm for insertion into an injection molding apparatus for the formation of a peripheral edge bead on the packet. The improved planarity and outer dimensional tolerances of the film packet assure not only that the film packet can be accurately positioned but also that the die-cut edge will be fully encapsulated with the application of the injection molded perimetric edge bead. The positioning of individual film packets against hard stop reference points by a Bernoulli effect apparatus results in a force being applied to the laminated perimetric edge of the film packet in a direction which is substantially parallel to the plane of the stiffening sheet. The laminated perimetric edge should have sufficient rigidity such that it will not deform when these forces are in the range of from 5 to 80 millinewtons (the level of force likely to result from using a Bernoulli effect device to position the dental packet against hard stop reference points).
Figure 1 is a cross-sectional view of a typical prior art dental film packet.
Figure 2 is a perspective view of the improved dental film packet of the present invention.
Figure 3 is a cross-sectional taken along line 3 - 3 of Figure 2.
Figure 4 is a perspective view of the Bernoulli positioning device used in the practice of the present invention.
Figure 5 is a top plan schematic of the dental film packet of Figure 2 residing in the Bernoulli positioning device shown in Figure 4.
Figure 6 is a side elevation/partial cross-sectional view of a robotic arm (in ghost) acquiring the dental film packet from the Bernoulli positioning device.
Figure 7 is a side elevation/partial cross-sectional view of a robotic arm placing the dental film packet into an injection mold base.
Figure 8 is a top plan view of the dental film packet residing in the injection mold base.
Figure 9 is a cross-sectional view of the dental film packet residing between the mold base and upper mold half.
Figure 10 is an enlarged view of the area within circle A of Figure 9.
Figure 11 is a perspective view of the improved dental film packet shown in Figure 2 with a comfort enhancing perimetric edge bead molded thereto.
Figure 12 is a cross-sectional view taken along line 12 - 12 of Figure 11.

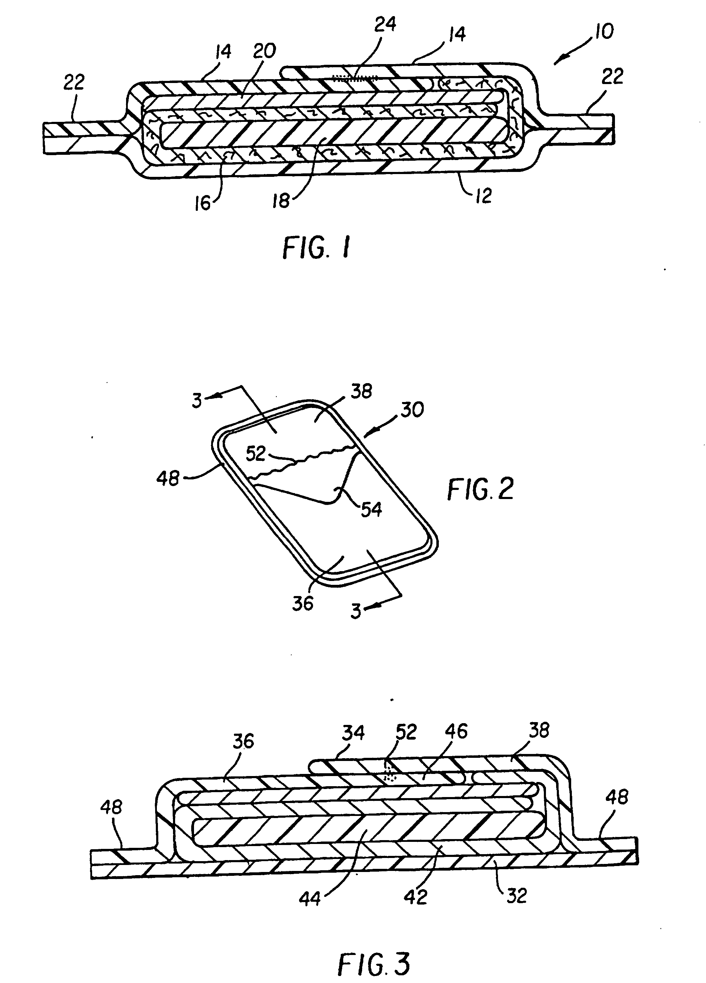

Turning first to Figure 1, there is shown a cross-section of a typical prior art dental film packet 10. Dental film packet 10 includes an outer envelope comprising a vinyl sheet 12 on one face of the dental film packet 10 and a pair of overlapping vinyl sheets 14 on the opposite face thereof. Contained between the 12 and overlapping sheets 14 are a paper wrap element 16, a film chip 18 and a lead foil 20. Vinyl sheets 12 and 14 project beyond dimensions of the paper wrap element 16, the film chip 18 and lead foil 20 to yield a perimetric edge 22. Laminated perimetric edge 22 allows for heat sealing of vinyl sheets 12 and 14 to one another to yield a light-tight perimeter to the dental film packet 10. In addition, a heat seal 24 is generated at the overlap of vinyl sheets 14 to provide an outer envelope which is completely light-tight and which is substantially watertight. This prior art dental film packet 10 therefore includes a relatively stiff and sharp perimetric edge created by the laminated perimetric edge 22. It is this relatively stiff and sharp perimetric edge which causes discomfort to the patient.

Turning next to Figures 2 and 3, there is shown the dental film packet 30 of the present invention. Dental film packet 30 includes an envelope comprising a first outer sheet or stiffening sheet 32 and an opposing second outer sheet 34. Second outer sheet 34 is actually comprised of a pair of overlapping sections 36 and 38. Outer sheets 32 and 34 are preferably made of a soft thermoplastic material such as, but not limited to polyvinyl chloride (PVC), ethylene vinyl acetate (EVA), or thermoplastic polyurethane (TPU), or a di-block polyamide copolymer. Sandwiched between outer sheets 32 and 34 are the typical elements found in a dental x-ray film packet. There is a paper wrap element 42, the film chip 44 and a lead foil 46. The dental x-ray film packet 30 is assembled by placing the paper wrap element 42, the film chip 44 and a lead foil 46 between outer sheets 32 and 34 and sealing the outer sheets 32 and 34 to one another to form a perimetric laminated edge 48. Perimetric laminated edge 48 can be formed by heat sealing, RF sealing, ultrasonic sealing, or any other sealing mechanism which can create a substantially airtight bond between outer sheets 32 and 34. There is also a transverse seal (indicated by line 52) affixing overlapping sections 36 and 38 together. Overlapping section 38 is preferably formed with a tab portion 54 which extends past transverse seal 52 to facilitate removal of the envelope for extraction and development of the film chip 44 after exposure.

Outer stiffening sheet 32 is preferably formed from a more rigid material than that of outer sheet 34. In this manner, the laminated perimetric edge 48 becomes substantially coplanar with outer stiffening sheet 32 as opposed to the perimetric edge of the prior art dental packet 10 shown in Figure 1, which is positioned more toward the center of the dental packet 10. This flatter (more planar) surface aids in the accurate positioning of the packet 30 in subsequent operations. Further, the more rigid material enhances the ability to die cut the packet 30 and maintain outside dimensions within tolerances. This enhances the ability to fit and accurately place the packet 30 in subsequent operations such as, for example, placement of the packet 30 in a comfort enhancing frame or applying a comfort enhancing bead thereto. This level of accuracy within dimensional tolerances is needed for automated assembly. As long as the material is more rigid, both outer sheets 32 and 34 forming the outer envelope of the packet 30 can be made of this more rigid material to garner the same benefits of positioning and dimensional control stated above.

One way of making outer stiffening sheet 32 more rigid is by reducing the amount of plasticizer used in the thermoplastic material of outer stiffening sheet 32 such that the thermoplastic material is less than 30 percent plasticizer. For example, in the case of polyvinyl chloride (PVC), the plasticizer could be dioctyl adipate (DOA), diethyl hexyl phthalate (DEHP) or dioctyl phthalate (DOP). The effect of reducing the amount of plasticizer is to increase the stiffness or modulus of the material from which outer stiffening sheet 32 is made so that during the heat sealing operation to form the perimetric laminated edge 48, the application of heat and the effects of the heating fixture do not as readily deform the material toward the center of the thickness of the packet 30 as was typical with the prior art packet 10.

The stiffening sheet 32 preferably has a tensile modulus in the range of from 700 to 28,000 Kg/cm². The envelope is formed by joining together the two outer plastic sheets at the marginal or peripheral area to form a perimetric laminated edge that preferably has a tensile modulus in the range of from 400 to 28,000 Kg/cm², and most preferably 15,700 Kg/cm². By way of example, if PVC is used as the material for outer sheets 32 and 34, then outer stiffening sheet 32 should have a thickness in the range of from 0.10 to 0.33 mm. This combination of thickness and tensile modulus will achieve the necessary rigidity for the perimetric laminated edge 48 to remain substantially coplanar with outer stiffening sheet 32. Further, this combination of thickness and tensile modulus when laminated to second outer sheet 34 results in a perimetric laminated edge 48 which will not deform under the forces applied thereto by a Bernoulli positioning apparatus, such forces preferably being in the range of from 5 to 80 millinewtons and which are in a direction that is substantially parallel to the plane of outer stiffening sheet 32. Again, by way of example, using an outer sheet 34 made of PVC with a nominal thickness of 0.20 mm and with a tensile modulus of 310 Kg/cm² in combination with an outer stiffening sheet 32 having a thickness of 0.20 mm and a tensile modulus of 15,700 Kg/cm² results in a perimetric laminated edge 48 with a thickness of 0.33 mm and a tensile modulus of 15,600 Kg/cm². This exemplary perimetric laminated edge 48 will not deform as result of a force in the range of from 5 to 80 millinewtons applied to the perimetric laminated edge 48 and parallel to the plane of outer stiffening sheet 32.

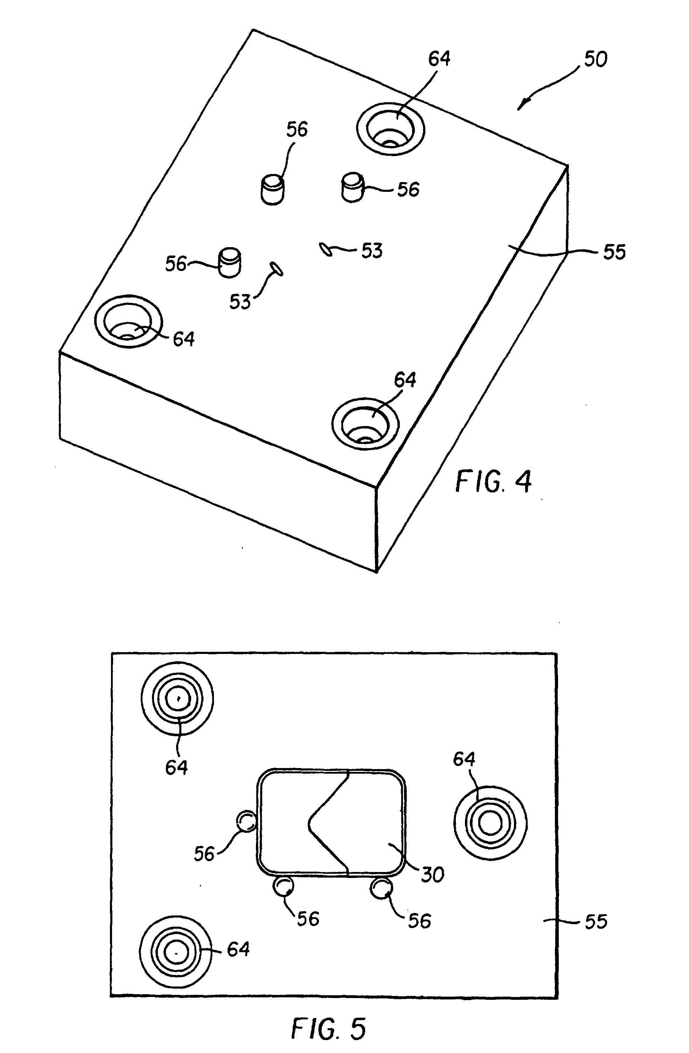

Looking next at Figure 4, the method of the present invention takes an intraoral dental radiographic film packet 30, and delivers it to a Bernoulli fixture 50. Delivery of the intraoral dental radiographic film packet 30 to a Bernoulli fixture 50 may be accomplished in a variety of ways such as, for example, a pick-and-place type mechanism with a vacuum cup used for packet acquisition and release. Application of the Bernoulli effect is used to orient and precisely position individual dental radiographic film packets 30. Compressed air is directed through a number of low angled air holes 53 in the top surface 55 of Bernoulli fixture 50. Air holes 53 are preferably inclined at an angle in the range of from 15° to 30° from horizontal, and most preferably at an angle of 25° from horizontal. The angle of inclination of air holes 53 can be shallower than 15° but machining of such shallow angled holes becomes more difficult. This creates a laminar flow of air along the surface 55. This airflow imparts a force on the dental packet 30, driving the dental packet 30 against the fixture reference features 56 which provide hard stops in two dimensions and thereby fixes the location of the dental packet 30 in a horizontal plane. Because of the airflow velocity, the pressure between the surface 55 of the Bernoulli fixture 50 and the packet 30 is lower than the pressure on the side of the packet 30 away from the Bernoulli fixture 50. Thus the packet 30 remains attracted to the surface 55, rather than being pushed away. As mentioned above, perimetric laminated edge 48 should not deform under the forces applied thereto by the Bernoulli fixture 50, such forces preferably being in the range of from 5 to 80 millinewtons and in a direction that is substantially parallel to the plane of outer stiffening sheet 32. There are preferably also three locating port s64 in top surface 55

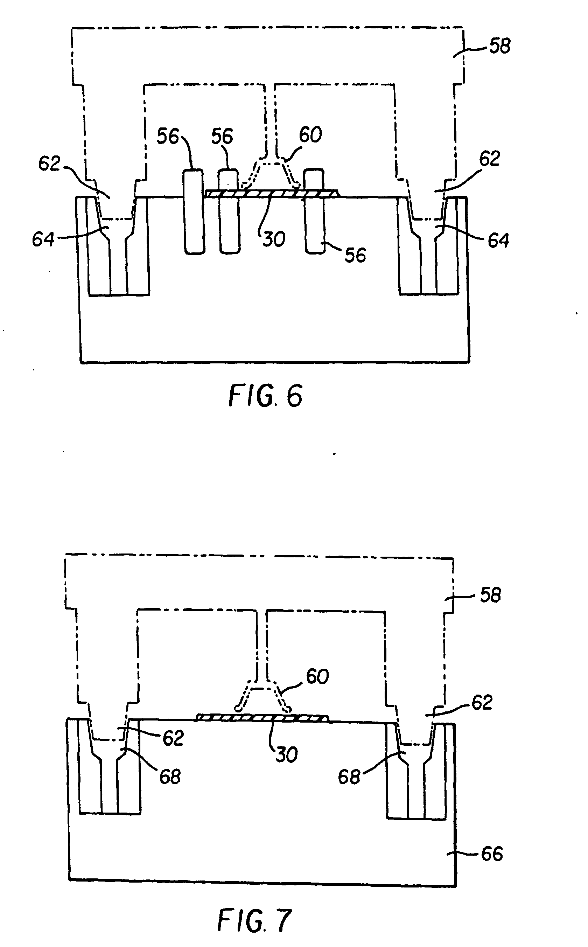

As shown schematically in Figure 5, with the packet 30 accurately oriented in a known position against fixture reference features 56, packet 30 can be picked up by a robotic arm 58 having a vacuum cup 60 disposed on the end thereof (see Figure 6). The robotic arm 58 may include locating pins 62 adapted to engage locating ports 64 in Bernoulli fixture 50 to ensure repeatable and precise acquisition of dental packets 30. Locating pins 62 engaging with locating ports 64 is just one method of ensuring repeatable and precise acquisition of dental packets 30. Other methods for repeatable and precise acquisition by a robotic arm are well known to those skilled in the art.

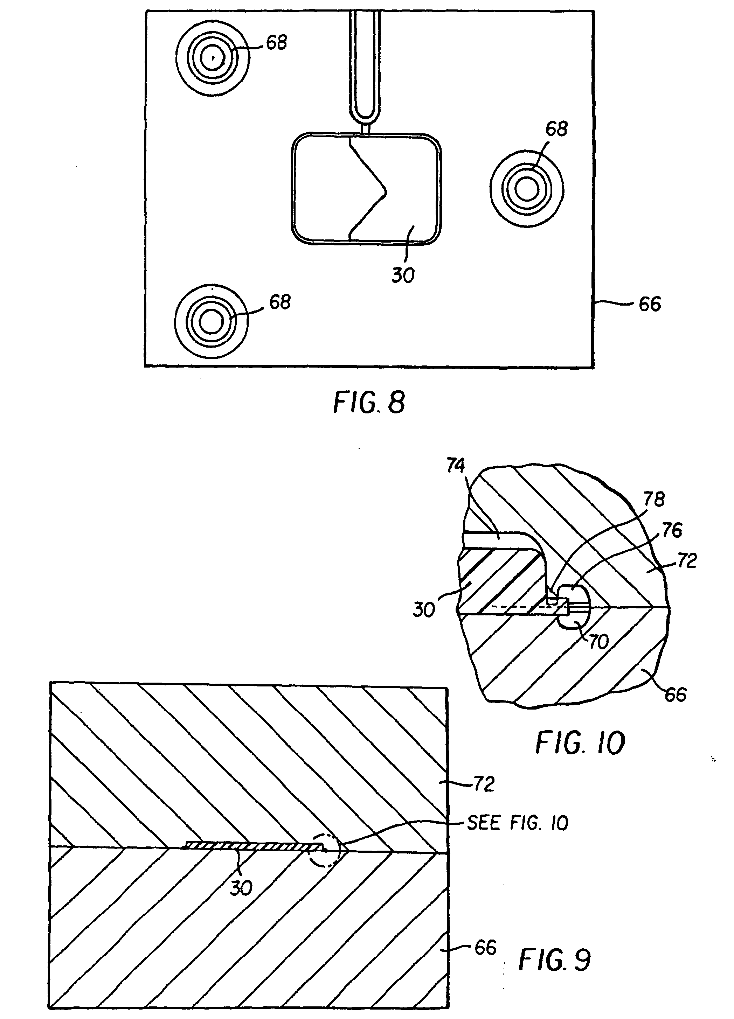

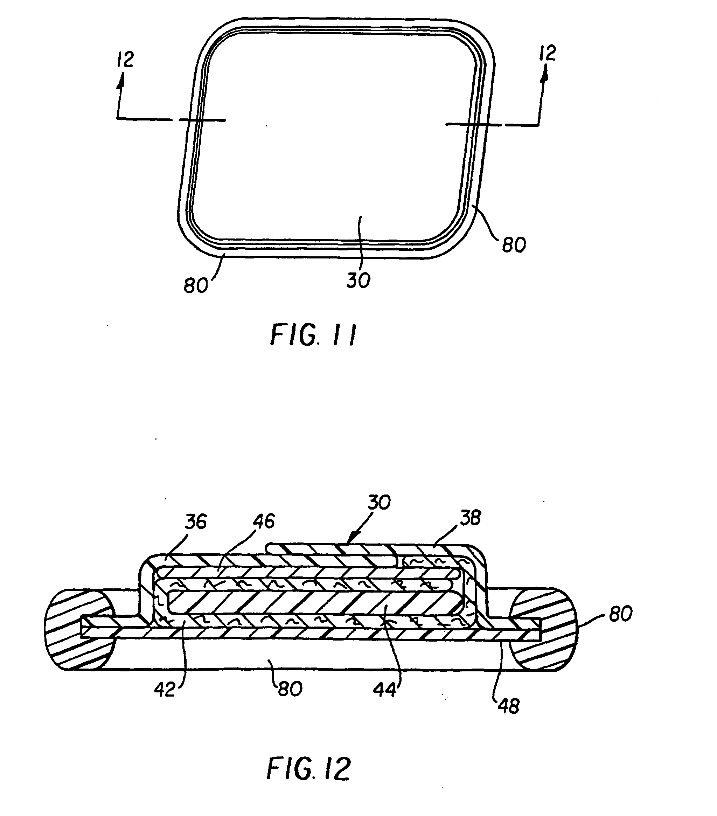

Once a dental film packet 30 has been acquired by vacuum cup 60 disposed on the end of robotic arm 58, the robotic arm 58 delivers the dental film packet 30 to an injection mold base 66 (see Figure 7). Injection mold base 66 includes locating ports 68 which, in conjunction with locating pins 62, ensure repeatable and precise placement of dental packets 30 into injection mold base 66 with reference to a lower bead canal 70 in the surface of injection mold base 66. With the dental film packet 30 so placed, vacuum pressure to vacuum cup 60 is discontinued and the robotic arm 58 leaves the dental film packet 30 on the injection mold base 66 as shown in Figure 8. An upper mold portion 72 is then placed in abutting position with injection mold base 66 as depicted in Figure 9. Upper mold portion 72 includes a packet cavity 74 in which a dental packet 30 resides (see Figure 10), and an upper bead canal 76. Packet cavity 74 and upper bead canal 76 are separated by a continuous lip element 78. With injection mold base 66 and upper mold portion 72 residing in abutting position, perimetric laminated edge 48 extends into the mold cavity formed by lower bead canal 70 and upper bead canal 76. A comfort-enhancing perimetric edge bead 80 (see Figures 11 and 12) can then be formed on perimetric laminated edge 48 by injection molding of a thermoplastic material into the mold cavity formed by lower bead canal 70 and upper bead canal 76. The perimetric edge bead 80 is comprised of a generally compliant material and is of a generally rounded cross-section so as to reduce the cutting sensation of the packet 30 by implying a softer, smoother, more rounded feel to the patient's gums, lips and inside of the oral cavity. A soft thermoplastic material such as, but not limited to, polyvinyl chloride (PVC) is preferably used for perimetric edge bead 80. The thermoplastic material preferably has a relatively low durometer material (e.g. 50 to 90 Shore A) to enhance the soft feeling. Other materials that can be used for injection molding of perimetric edge bead 80 include, for example, thermoplastic elastomers and di-block polyamide copolymers.

It should be clear to those skilled in the art that the ability to accurately position a packet 30 in a Bernoulli fixture 50 by driving it against hard stop reference features 56 is affected by the flexibility of the marginal area (perimetric laminated edge 48). The added rigidity of outer sheet 32 should be great enough to resist deformation under the forces applied by the Bernoulli fixture 50. In addition, the added rigidity and planarity of the marginal area (perimetric laminated edge 48) ensures residence of the perimetric laminated edge 48 in the mold cavity formed by lower bead canal 70 and upper bead canal 76. This, in turn, ensures encapsulation of the die-cut perimetric edge of the packet 30 which allows for the application of the comfort-enhancing perimetric edge bead 80 which encapsulates at least part of the sharp edge of the die-cut perimetric edges of the perimetric laminated edge 48.

Although stiffening sheet 32 has been described herein as being an outer sheet it should be apparent to those skilled in the art that an extra or third sheet could be applied outside the stiffening sheet 32. This extra or third sheet would however add to expense and bulk of each packet 30. Thus for the purposes of this application the term "outer sheet" includes an envelope forming sheet in which the paper wrap element 42, the film chip 44 and a lead foil 46 reside and which forms at least in part the laminated perimetric edge 48.

## Claims

1. An intraoral x-ray film packet comprising:
(a) a first outer sheet having a tensile modulus of at least 700 Kg/cm²;
(b) a second outer sheet;
(c) a film chip residing between said first outer sheet and said second outer sheet; and
(d) a continuous perimetric seal affixing said first outer sheet and said second outer sheet and forming a perimetric laminated edge, said perimetric laminated edge being substantially coplanar with said first outer sheet.

2. An intraoral x-ray film packet as recited in claim 1 wherein:
said first outer sheet has a tensile modulus in the range of from 700 to 28,000 Kg/cm².

3. An intraoral x-ray film packet as recited in claim 1 wherein:
said perimetric laminated edge will not deform when subjected to a force of not more than 80 millinewtons in a direction which is substantially parallel to the plane in which said first outer sheet resides.

4. An intraoral x-ray film packet as recited in claim 1 wherein:
said perimetric laminated edge has a tensile modulus in the range of from 400 to 28,000 Kg/cm².

5. An intraoral x-ray film packet as recited in claim 1 wherein:
said first and second outer sheets are made from a thermoplastic material.

6. An intraoral x-ray film packet as recited in claim 5 wherein:
said first outer sheet is made more rigid than said second outer sheet by reducing the amount of plasticizer in said thermoplastic material.

7. A method for making an intraoral x-ray film packet comprising the steps of:
(a) forming a first outer sheet from a thermoplastic material having a tensile modulus in the range of from 700 to 28,000 Kg/cm² ;
(b) forming a second outer sheet from a thermoplastic material; and
(c) sealing the first outer sheet and the second outer sheet together to form an envelope with a laminated perimetric edge, the envelope containing a film chip, the laminated perimetric edge being substantially coplanar with the first outer sheet.

8. A method as recited in claim 7 wherein:
said sealing step results in the perimetric laminated edge having a tensile modulus in the range of from 400 to 28,000 Kg/cm².

9. A method as recited in claim 8 wherein:
said sealing step results in the perimetric laminated edge being able to resist deformation when subjected to a force of at least 5 millinewtons and not more than 80 millinewtons in a direction which is substantially parallel to the plane in which the first outer sheet resides.

10. A method as recited in claim 7 wherein the second outer sheet is comprised of a thermoplastic material containing more than 30% plasticizer.

## Patentansprüche

1. Intraorale Röntgenfilmpackung mit:
a) einer ersten äußeren Folie, die ein Zugmodul von mindestens 700 kg/cm² aufweist;
b) einer zweiten äußeren Folie;
c) einem zwischen der ersten und der zweiten äußeren Folie vorgesehenen Filmchip; und
d) einer durchgehenden perimetrischen Dichtung, die die erste äußere Folie mit der zweiten äußeren Folie verbindet, wodurch eine perimetrisch laminierte Kante entsteht, die im wesentlichen koplanar mit der ersten äußeren Folie verläuft.

2. Intraorale Röntgenfilmpackung nach Anspruch 1, worin:
die erste äußere Folie ein Zugmodul im Bereich von 700 bis 28,000 kg/cm² aufweist.

3. Intraorale Röntgenfilmpackung nach Anspruch 1, worin:
die perimetrisch laminierte Kante sich nicht verformt, wenn eine Kraft von höchstens 80 Millinewton in einer Richtung auf sie wirkt, die im wesentlichen parallel zur Ebene verläuft, in der die erste äußere Folie liegt.

4. Intraorale Röntgenfilmpackung nach Anspruch 1, worin:
die perimetrisch laminierte Kante ein Zugmodul im Bereich von 400 bis 28,000 kg/cm² aufweist.

5. Intraorale Röntgenfilmpackung nach Anspruch 1, worin:
die erste und zweite äußere Folie aus einem thermoplastischen Material bestehen.

6. Intraorale Röntgenfilmpackung nach Anspruch 5, worin:
die erste äußere Folie durch Reduzierung der Menge an Weichmacher im thermoplastischen Material steifer ist als die zweite äußere Folie.

7. Verfahren zum Herstellen einer intraoralen Röntgenfilmpackung, mit den Schritten:
a) Ausbilden einer ersten äußeren Folie aus einem thermoplastischen Material mit einem Zugmodul im Bereich von 700 bis 28,000 kg/cm².
b) Ausbilden einer zweiten äußeren Folie aus einem thermoplastischen Material; und
c) Verschweißen der ersten und der zweiten äußeren Folie, um eine Hülle mit einer laminierten perimetrischen Kante zu bilden, wobei die Hülle einen Filmchip aufweist und die laminierte perimetrische Kante im wesentlichen koplanar mit der ersten äußeren Folie verläuft.

8. Verfahren nach Anspruch 7, worin:
der Schritt des Verschweißens dazu führt, dass die perimetrisch laminierte Kante ein Zugmodul im Bereich von 400 bis 28,000 kg/cm² aufweist.

9. Verfahren nach Anspruch 8, worin:
der Schritt des Verschweißens zu einer perimetrisch laminierten Kante führt, die einer Verformung widersteht, wenn sie einer Kraft ausgesetzt ist von mindestens 5 Millinewton und höchstens 80 Millinewton in einer Richtung, die im wesentlichen koplanar zur Ebene verläuft, in der die erste äußere Folie liegt.

10. Verfahren nach Anspruch 7, worin die zweite äußere Folie aus einem thermoplastischen Material besteht, das mehr als 30% Weichmacher enthält.

## Revendications

1. Conditionnement de film radiographique intraoral comprenant :
(a) une première feuille extérieure présentant un module de traction d'au moins 700 kg/cm²,
(b) une seconde feuille extérieure,
(c) un morceau de film se trouvant entre ladite première feuille extérieure et ladite seconde feuille extérieure, et
(d) un joint périmétrique continu attachant ladite première feuille extérieure et ladite seconde feuille extérieure et formant un bord stratifié périmétrique, ledit bord stratifié périmétrique étant pratiquement coplanaire avec ladite première feuille extérieure.

2. Conditionnement de film radiographique intraoral selon la revendication 1, dans lequel :
ladite première feuille extérieure présente un module de traction dans la plage de 700 à 28 000 kg/cm².

3. Conditionnement de film radiographique intraoral selon la revendication 1, dans lequel :
ledit bord stratifié périmétrique ne se déforme pas lorsqu'il est soumis à une force qui n'est pas supérieure à 80 millinewtons dans une direction qui est pratiquement parallèle au plan dans lequel se trouve ladite première feuille extérieure.

4. Conditionnement de film radiographique intraoral selon la revendication 1, dans lequel :
ledit bord stratifié périmétrique présente un module de traction dans la plage de 400 à 28 000 kg/cm².

5. Conditionnement de film radiographique intraoral selon la revendication 1, dans lequel :
lesdites première et seconde feuilles extérieures sont constituées d'une matière thermoplastique.

6. Conditionnement de film radiographique intraoral selon la revendication 5, dans lequel :
ladite première feuille extérieure est réalisée de façon plus rigide que ladite seconde feuille extérieure en réduisant la quantité de plastifiant dans ladite matière thermoplastique.

7. Procédé de fabrication d'un conditionnement de film radiographique intraoral comprenant les étapes consistant à :
(a) former une première feuille extérieure à partir d'une matière thermoplastique présentant un module de traction dans la plage de 700 à 28 000 kg/cm²,
(b) former une seconde feuille extérieure à partir d'une matière thermoplastique, et
(c) sceller ensemble la première feuille extérieure et la seconde feuille extérieure pour former une enveloppe présentant un bord périmétrique stratifié, l'enveloppe contenant un morceau de film, le bord périmétrique stratifié étant pratiquement coplanaire avec la première feuille extérieure.

8. Procédé selon la revendication 7, dans lequel :
ladite étape de scellement résulte en un bord stratifié périmétrique présentant un module de traction dans la plage de 400 à 28 000 kg/cm².

9. Procédé selon la revendication 1, dans lequel :
ladite étape de scellement résulte en un bord stratifié périmétrique qui peut résister à une déformation lorsqu'il est soumis à une force d'au moins 5 millinewtons et qui n'est pas supérieure à 80 millinewtons dans une direction qui est pratiquement parallèle au plan dans lequel se trouve la première feuille extérieure.

10. Procédé selon la revendication 7, dans lequel ladite seconde feuille extérieure est constituée d'une matière thermoplastique contenant plus de 30 % de plastifiant.
